# EUROPÄISCHE PATENTSCHRIFT

(11) **EP 3 612 315 B1**
(45) Veröffentlichungstag und Bekanntmachung des Hinweises auf die Patenterteilung: **30.03.2022**
(21) Anmeldenummer: 18719154.9
(22) Anmeldetag: 19.04.2018
(51) Int. Cl.: B05B 5/00, B05B 5/16, A45D 34/00

(54) **FLÜSSIGKEITSTANK FÜR EINEN ZERSTÄUBER**
LIQUID TANK FOR A NEBULIZER
RÉSERVOIR DE LIQUIDE POUR PULVÉRISATEUR

(30) Priorität: 21.04.2017 DE 102017108615
(43) Veröffentlichungstag der Anmeldung: 26.02.2020
(73) Patentinhaber: J. Wagner GmbH, 88677 Markdorf (DE)
(72) Erfinder: BARTHELMES, Jan, 88682 Salem (DE); JELTSCH, Thomas, 88048 Friedrichshafen (DE); STOHL, Holger, 88677 Markdorf (DE)
(74) Vertreter: Otten, Roth, Dobler & Partner mbB Patentanwälte
(86) Internationale Anmeldenummer: PCT/EP2018/060122
(87) Internationale Veröffentlichungsnummer: WO 2018/193070

(56) Entgegenhaltungen:
- WO-A1-2011/037112
- WO-A2-2005/072059
- GB-A- 2 061 769
- JP-A- 2008 212 857
- US-A- 4 612 598
- US-A- 5 221 050
- US-A1- 2007 152 086
- US-A1- 2009 200 392
- US-B1- 6 267 274

## Beschreibung

Die Erfindung betrifft einen Flüssigkeitstank für einen Zerstäuber.

Zerstäuber, insbesondere im Bereich der Haushaltswaren und der Kosmetik sind in der Regel handbetrieben oder werden durch motorisiert aufgebrachte Druckunterschiede betätigt.

Aus der US 4,612,598 A1 ist eine elektrostatische Sprühvorrichtung bekannt, bei welcher die Flüssigkeitszufuhr zur Sprühdüse über ein mechanisches Ventil erfolgt, das von einem Betätigungselement betätigt wird. Das Betätigungselement ist mit einem Fernbedienungshebel, der vorzugsweise elektrisch leitend ist, über ein Verbindungselement verbunden, das einen elastisch dehnbaren Abschnitt enthält. Das Verbindungselement ist elektrisch leitend und stellt somit eine elektrische Verbindung vom Hebel zum Sprühkopf her.

Weiterhin ist aus der GB 2 061 769 A eine Vorrichtung zum elektrostatischen Sprühen bekannt, welche eine Stromversorgung, einen Hochspannungsgenerator, eine Sprühdüse, von der mindestens ein Teil der Oberfläche elektrisch leitend ist, eine Elektrode, die neben der Düse angeordnet und von dieser isoliert ist, mit elektrischen Anschlüssen zum Anschließen der Stromversorgung zu den Eingangsanschlüssen des Generators und der Elektrode zu einem Ausgangsanschluss des Generators und der Düse zu dem anderen Ausgangsanschluss des Generators bekannt, wobei ein Behälter eine Öffnung zum Zuführen von Flüssigkeit aufweist. Hierbei kann ein Verschluss eine abnehmbare Kappe, ein Kugelventil, das mit einem Finger an den Befestigungsmitteln betätigt wird oder ein Ventil sein, das durch elektrostatische Abstoßung betätigt wird.

Aus der WO 2005/072059 A2 ist eine Vorspannungsfunktionalität für einen Spender, die ein Fluid als Reaktion auf Temperaturänderungen intermittierend abgibt, bekannt, wobei diese einen Kolben umfasst, wobei diese ein temperaturempfindliches Schaltelement, das als Reaktion auf Temperaturänderungen verschiebbar ist und auf dem Kolben montiert ist, umfasst und wobei diese ein Federspannungselement umfasst, das in den Kolben eingreift, um das Schaltelement bei einer ausgewählten Temperatur wesentlich zu verschieben.

Aus der US 6 267 274 B1 ist eine Vorrichtung zum selektiven Erhöhen der Feuchtigkeit in einer vorbestimmten Zone bekannt, wobei die Vorrichtung ein handgehaltener Einweg-Druckbehälter vom Aerosoltyp ist, der einen handgehaltenen Einwegbehälter vom Aerosoltyp umfasst, der eine Aerosolzusammensetzung umfasst, die im Wesentlichen aus Wasser und einem flüssigen Treibmittel in dem Behälter besteht, der ein Ventil umfasst und der einen Aktuator umfasst.

Weiterhin ist aus der WO 2005/072059 A2 ein Zerstäuber mit einem Thermometer zur Messung der Umgebungstemperatur bekannt.

Die zu zerstäubenden Medien, in der Regel Flüssigkeiten werden dabei in Flüssigkeitstanks an dem Zerstäuber bereitgestellt. Nach der Entleerung eines Flüssigkeitstanks wird dieser ausgetauscht und gegen einen Tank mit neuer Füllung ersetzt. Der Flüssigkeitstank wird sodann entsorgt oder einem Recycling-System zugeführt.

Nachteilig ist, dass entsprechende Systeme keinen Austausch unterschiedlicher Flüssigkeiten erlauben, welche an dem Zerstäuber zur Verfügung stehen sollen.

Aufgabe der Erfindung ist es, einen Flüssigkeitstank so zu modifizieren, dass eine Schädigung des Inhalts durch Umwelteinflüsse erkennbar ist.

Die Erfindung ist im beigefügten Anspruchssatz beschrieben. Die Erfindung geht von einem Flüssigkeitstank für einen Zerstäuber für Flüssigkeiten, insbesondere einen elektrostatischen Zerstäuber aus, wobei der Flüssigkeitstank einen Aufnahmeraum für eine Flüssigkeit und eine Begrenzungswand zur Begrenzung des Aufnahmeraums gegenüber der Umgebung umfasst. Hierbei umfasst der Flüssigkeitstank ein selbst schließendes Ventil zum Anschluss am Zerstäuber.

Elektrostatische Zerstäubung umfasst im Sinne der vorliegenden Erfindung alle Zerstäubungsvorgänge, welche Flüssigkeiten mit Effekten der Einwirkung einer Hochspannung zerstäuben. Insbesondere auch elektrohydrodynamische Effekte sowie elektrokinetische Effekte sind von dem Begriff dieser Art von Zerstäubung umfasst. Im Sinne der Erfindung kann unter einer elektrostatischen Zerstäubung auch eine elektrohydrodynamische Zerstäubung aufgefasst werden.

Durch die Verwendung eines selbst schließenden Ventils kann der Flüssigkeitstank vom Zerstäuber auch ohne vollständige Entleerung entnommen werden, ein anderer Flüssigkeitstank kann angeschlossen werden und ein Austausch der Flüssigkeiten je nach Anwendungsfall ist ermöglicht. Insbesondere bietet sich dies für sogenannte elektrostatische Zerstäuber an, welche Gegenstand von Erfindungen der Anmelderin sind. Insbesondere bei elektrostatischen Zerstäubern, welche zur Aufbringung unterschiedlicher Flüssigkeiten, wie neben Farben oder Lacken auch z.B. in besonderen Bauformen für Kosmetika geeignet sind, ist eine flexible Austauschbarkeit von z.B. unterschiedlichen Kosmetikeigenschaften oder Anwendungsspezifikationen unentbehrlich.

Der Flüssigkeitstank wird über das Ventil an einen Zerstäuber angeschlossen und an dessen Leitungssystem kontaktiert und eine Abgabe von Flüssigkeit wird bei kontaktiertem Ventil durch Öffnung des Ventils ermöglicht. Sobald der Flüssigkeitstank vom Zerstäuber abgenommen wird, verschließt sich das Ventil von selbst. Eine zusätzliche Abdeckung, z.B. ein Schraubverschluss ist nicht erforderlich, kann aber ergänzend vorgesehen sein.

In einer bevorzugten Ausführung ist das selbst schließende Ventil als Membranventil, als Lippenventil, als Kugelventil oder Federventil ausgebildet.

Derartige Ventile weisen üblicherweise eine Ruhestellung auf, in welcher Sie ohne Einwirkung äußerer mechanischer Mittel, insbesondere Öffnungsmittel, geschlossen sind. Im Einzelfall kann, z.B. bei einem Kugelventil oder einem Lippenventil, die Dichtwirkung auch durch eine Druckbeaufschlagung vom Inneren des Aufnahmeraums erfolgen, so dass zur Freigabe der Flüssigkeit bei Anschluss an einen Zerstäuber die Kugel gegen diesen Druck entgegen der Strömungsrichtung der zu entnehmenden Flüssigkeit bewegt werden muss. Ähnlich können Membranventile oder Federventile beaufschlagt sein.

Ein Lippenventil erlaubt eine Strömung in eine Richtung durch in Strömungsrichtung fluchtend angeordnete Ventillippen. Bei Fluss entgegen der Strömungsrichtung werden diese Ventillippen aneinander gepresst und der Querschnitt wird bis zum dichtenden Abschluss verengt. Kombinationen von derartigen Ventilen erlauben auch unterschiedliche Flusswege bei unterschiedlichen Flussrichtungen, z.B. eine Abzweigung bei Rückfluss in einen Entsorgungsraum für überschüssiges Material, welches nicht in den Flüssigkeitstank zurück gelangen soll.

In einer weiteren Ausführungsform ist vorgesehen, dass die Begrenzungswand und/oder ein die Begrenzungswand umgebendes Gehäuse aus isolierendem Material besteht, insbesondere eine elektrostatische Abschirmung ausbildet wobei das Material bevorzug eine Durchschlagfestigkeit von mindestens 30kV besitzt und/oder insbesondere eine Wandstärke von mindestens 1 mm aufweist.

Bei elektrostatischen Zerstäubern findet in der Regel eine Hochspannung Anwendung, welche u.a. auch die bevorratete Flüssigkeit aufladen kann. Die zur Zerstäubung erforderliche Hochspannung liegt aufgrund der in der Regel zumindest leicht leitfähigen Flüssigkeit auch an der Flüssigkeit im Flüssigkeitstank an. Somit ist eine Isolierung gegenüber dem Außenraum erforderlich, um einen elektrischen Schlag bzw. eine elektrostatische Entladung am Benutzer zu vermeiden. Besonders bevorzugt kann dabei Polypropylen, ABS, PTFE, Polyethylene oder Polyester als Gehäusematerial zum Einsatz kommen.

Bevorzugt kann das Gehäuse auch eine Belüftungsöffnung enthalten, um z.B. den Innenraum gegenüber dem Außenraum zu belüften.

In einer Weiterbildung kann vorgesehen sein, dass die Begrenzungswand und/oder ein die Begrenzungswand umgebendes Gehäuse eine Kontaktierung auf ein Bezugspotential gegenüber einer Hochspannung, z.B. gemeinsamer Minuspol einer Hochspannungsquelle und eines Akkus, umfasst, wobei die Kontaktierung zumindest über einen Kontakt zur Verbindung mit dem elektrostatischen Zerstäuber verfügt. Das Gehäuse kann hierzu insbesondere eine leitfähige Außenschicht umfassen, welche an die Kontaktierung angebunden ist.

Durch die Kontaktierung kann ergänzend oder alternativ dafür gesorgt werden, dass die oben beschriebene elektrostatische Entladung am Benutzer vermieden wird.

In einer besonderen Ausführungsform ist an dem Flüssigkeitstank mindestens ein Mittel zur Identifizierung (Kodierung) des Flüssigkeitstanks umfasst, wobei dieses Mittel insbesondere als mechanische Kodierung, bevorzugt zur Ausbildung eines Formschlusses, und/oder elektronische Kodierung, insbesondere zur Übermittlung von Informationen über den Flüssigkeitstank, und/oder elektrische Kodierung, insbesondere zur Ausbildung von elektrischen Leitungswegen über Kontakte, ausgebildet sind.

Die Mittel zur Identifizierung können insbesondere als RFID Markierung oder dergleichen ausgebildet sein, wobei durch den Zerstäuber, insbesondere dessen Kontrollelektronik die RFID Markierung sowohl gelesen als auch ganz oder teilweise beschrieben werden kann. Letzteres ist insbesondere dann vorteilhaft, wenn Gebrauchsdaten wie z.B. das Datum der ersten Öffnung des Flüssigkeitstanks oder Daten über die entnommene Flüssigkeitsmenge gespeichert werden sollen. Dadurch wird die Austauschbarkeit des Flüssigkeitstanks optimiert.

Durch Mittel zur Identifizierung kann ein Geräteparameter an einem Zerstäuber vorgewählt werden. Zudem können möglicherweise nicht zur Anwendung vorgesehene Flüssigkeitstanks identifiziert und deren Anwendung vermieden werden. Auch die ggf. erforderliche Reinigung eines Zerstäubers kann durch eine Identifikation des Flüssigkeitstanks dem Benutzer signalisiert werden, um ein gewünschtes Ergebnis zu erhalten. Nach einer Anwendung von Kosmetika sollte z.B. das Gerät gereinigt werden, bevor ein Flüssigkeitstank mit Deodorant eingesetzt wird. Darauf kann das Gerät bei Identifikation der Flüssigkeitstanks hinweisen.

Die mechanische Kodierung kann z.B. durch Bohrungen, Vorsprünge oder Rasten realisiert sein. Eine elektronische Kodierung, z.B. über Widerstände, einen Identifikationschip oder eine RFID kann zusätzliche Informationen enthalten, welche z.B. ein Verfallsdatum oder dergleichen bereitstellen. Eine rein elektrische Kodierung kann z.B. durch die Bereitstellungen von Leitungswegen oder elektrischen Brücken erfolgen.

In einer besonders bevorzugten Ausführung ist vorgesehen, dass die Begrenzungswand flexibel, insbesondere als kollabierbarer Beutel ausgebildet ist oder dass die Begrenzungswand als Zylinder, vorzugsweise mit einem passiv geführten Folgekolben bzw. Kolbenboden ausgebildet ist.

Eine selbst kollabierende Ausführung der Begrenzungswand bietet den Vorteil, dass eine Entleerung ohne zusätzliche mechanische Belüftung eines Aufnahmeraums ermöglicht ist. Auch die Möglichkeit eines passiven Folgekolbens erlaubt eine Entleerung ohne zusätzliche mechanische Einwirkung oder Öffnung des Aufnahmeraums. Die bevorratete Flüssigkeit ist damit stets gegenüber der Umgebung versiegelt und kann den entsprechenden Anforderungen, z.B. an die Hygiene damit genügen.

Bevorzugt kann am erfindungsgemäßen Flüssigkeitstank mindestens ein weiterer Aufnahmeraum für mindestens eine weitere Flüssigkeit umfasst sein. Dabei ist eine weitere Begrenzungswand zur Begrenzung des Aufnahmeraums gegenüber der Umgebung und dem ersten Aufnahmeraum ebenso vorgesehen.

Ein weiterer Aufnahmeraum ermöglicht die Bereitstellung von mehreren Komponenten in einem Flüssigkeitstank. So kann z.B. eine Farbe durch Einstellung eines Mischungsverhältnisses aus zwei Farbtönen oder ggf. ein kosmetisches Material durch Mischung von Trägerflüssigkeit und Kosmetikanteil auf einen gewünschten Erscheinungs- oder Wirkeffekt abgestimmt werden. Auch andere Varianten von mehreren Komponenten sind denkbar.

In einer bevorzugten Ausführungsform ist vorgesehen, dass mindestens eine Füllstandanzeige zur Anzeige des Füllstands der Flüssigkeit in dem Aufnahmeraum umfasst ist.

Die Füllstandanzeige kann mechanisch, z.B. durch ein Sichtfenster oder elektronisch, z.B. durch einen Volumenstromsensor realisiert werden. Auch andere Arten der Erfassung der entnommenen Flüssigkeitsmenge sind denkbar, z.B. über eine Protokollierung von Pumpenparametern wie Laufzeit oder Umdrehungen des Pumpenmotors bei Kenntnis der Pumpengeometrie.

Erfindungsgemäß ist es vorgesehen, dass mindestens ein Sensorelement zur Erfassung der Umgebungsparameter des Flüssigkeitstanks und/oder der Flüssigkeit umfasst ist, wobei das Sensorelement über ein Speicherelement zur Speicherung der Umgebungsparameter verfügt.

Durch einen Sensor, z.B. einen Temperatursensor oder dergleichen, kann eine Einwirkung der Umgebungsparameter auf die Flüssigkeit erfasst werden. Wird diese z.B. zu heiß oder zu kalt gelagert, kann die Flüssigkeit denaturieren, was dem Benutzer angezeigt wird. Durch die optionale Verwendung eines Speicherelements kann diese Information verarbeitet und/oder später bereitgestellt oder ergänzt werden. Auch eine Verknüpfung von Sensorelementen ist denkbar, z.B. zwischen einem Füllstandsensor und einem Zeitzähler seit der ersten Öffnung des Flüssigkeitsbehälters.

Die Sensordaten können auch an einen Zerstäuber über eine Schnittstelle übergeben werden, welcher sodann Auswertungen vornimmt oder die Daten zur weiteren Verwendung speichert. Eine entsprechende Schnittstelle wird in einer bevorzugten Ausführungsform von dem erfindungsgemäßen Flüssigkeitstank ebenfalls umfasst.

In einer Ausgestaltung der Erfindung sieht der Flüssigkeitstank im Umgebungsbereich des Ventils an einem Gehäuse ein erstes mechanisches Kupplungselement, insbesondere ein Bajonettelement oder einen Drehriegelmechanismus vor. Auf diese Weise kann der Flüssigkeitstank einfach und sicher am Zerstäuber angeordnet und prozesssicher befestigt werden. Bevorzugt ist zusätzlich an einem dem Ventil abgewandten Abschnitt des Gehäuses ein zweites mechanisches Kupplungselement umfasst, dessen Geometrie korrespondierend verbindbar zum ersten mechanischen Kupplungselement ausgebildet ist. Auf diese Weise können die Flüssigkeitstanks miteinander gekoppelt werden. Dies dient z.B. einer vereinfachten Lagerung oder kann bei entsprechend stabiler Ausbildung der Kupplungselemente zur Verlängerung z.B. eines Handgriffs dienen. Auch der Flüssigkeitstank selbst kann am Zerstäuber als Handgriff oder zumindest als Teil des Handgriffs dienen.

Im Sinne der Erfindung kann unter einer elektrostatischen Zerstäubung auch eine elektrohydrodynamische Zerstäubung aufgefasst werden.

Im Sinne der Erfindung ist unter einer Flüssigkeit jedwedes Liquid aufzufassen. Im Sinne der Erfindung ist insbesondere vorgesehen, dass die Flüssigkeit ein Kosmetikum ist. Die Flüssigkeit kann auch eine flüssige Farbe oder Lack oder dergleichen sein.

In den nachfolgenden Figuren ist eine exemplarische Ausführungsform des erfindungsmäßen Flüssigkeitsbehälters schematisch dargestellt. Dabei zeigen
- Fig. 1: einen ersten Flüssigkeitstank in perspektivischer Darstellung mit Ventil;
- Fig. 2: einen Flüssigkeitstank in geschnittener Darstellung mit Ventil und flachem Boden;
- Fig. 3: einen Flüssigkeitstank in geschnitten explodierter Darstellung mit Ventil und Kupplungselement am Boden;
- Fig. 4: einen zweiten Flüssigkeitstank in geschnitten explodierter Darstellung mit Ventil und Kupplungselement und Passiv-Folgekolben;

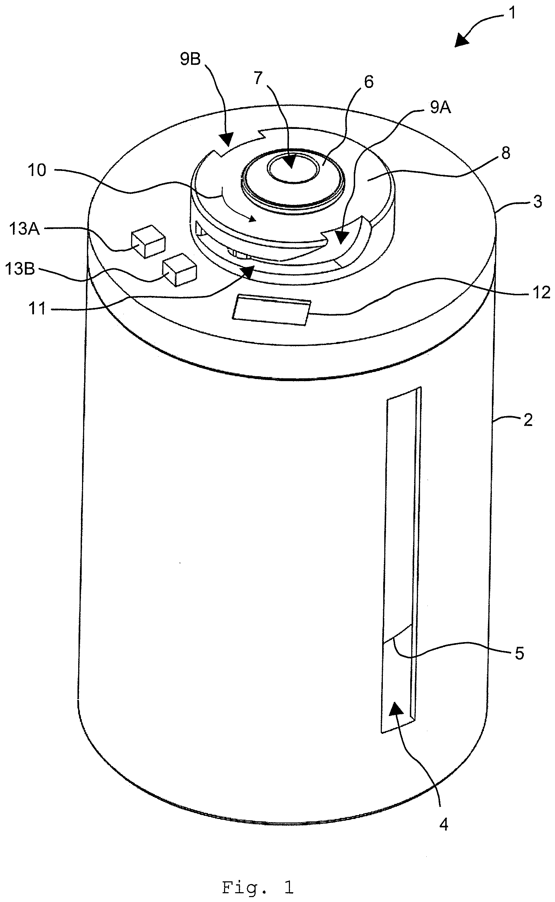

Im Einzelnen zeigt Fig. 1 eine erste Ausführung eines Flüssigkeitstanks 1. Der Flüssigkeitstank 1 umfasst dabei ein Gehäuse 2 sowie einen Gehäusedeckel 3. Das Gehäuse 2 besitzt an einer Seite eine Öffnung 4 durch welche ein in dem Gehäuse 2 angeordneter Beutel 5 zur Aufnahme der Flüssigkeit sichtbar ist. Durch die Öffnung 4 kann der Füllstand des Beutels 5 abgelesen werden.

Am Gehäusedeckel 3 ist zentrisch ein Ventil 6, vorliegend in der Form eines Lippenventils angeordnet. Das Ventil 6 weist zentrisch eine Eintrittsöffnung 7 für ein Öffnungsmittel (nicht dargestellt) auf, welches an einem Zerstäuber vorgesehen ist, und bei eingesetztem Flüssigkeitstank 1 in die Eintrittsöffnung 7 eintritt.

Das Ventil 6 umgebend ist ein Kupplungsmittel 8 angeordnet, wobei das Kupplungsmittel 8 vorliegend als Drehkupplung ausgebildet ist. Dabei greift ein am Zerstäuber angeordneter Vorsprung in die Nuten 9A, 9B ein und wird durch eine Drehbewegung in Richtung 10 entlang des Kanals 11 formschlüssig eingerastet. Auf diese Weise ist eine einfache, selbst zentrieren den Anordnung des Flüssigkeitstanks 1 an einem Zerstäuber sichergestellt. Der Gehäusedeckel 3 umfasst weiterhin Vorrichtungen zur mechanischen und/oder elektrischen bzw. elektronischen Kodierung. Eine mechanische Kodierung kann beispielsweise durch eine Rastöffnung 12 realisiert werden, in welche eine Rastklinke des Zerstäuber eingerastet. Eine elektrische Kodierung kann durch die Bereitstellung einer leitfähigen Brücke, z.B. einen leitfähigen Gehäusedeckel 3 erzielt werden. Für eine elektronische Kodierung können in einem oder mehreren Elementen 13A, 13B Informationen auf einem Chip oder anderen Datenträgern bereitgestellt werden. Auch drahtlose Informationen, z.B. eine RFID sind denkbar.

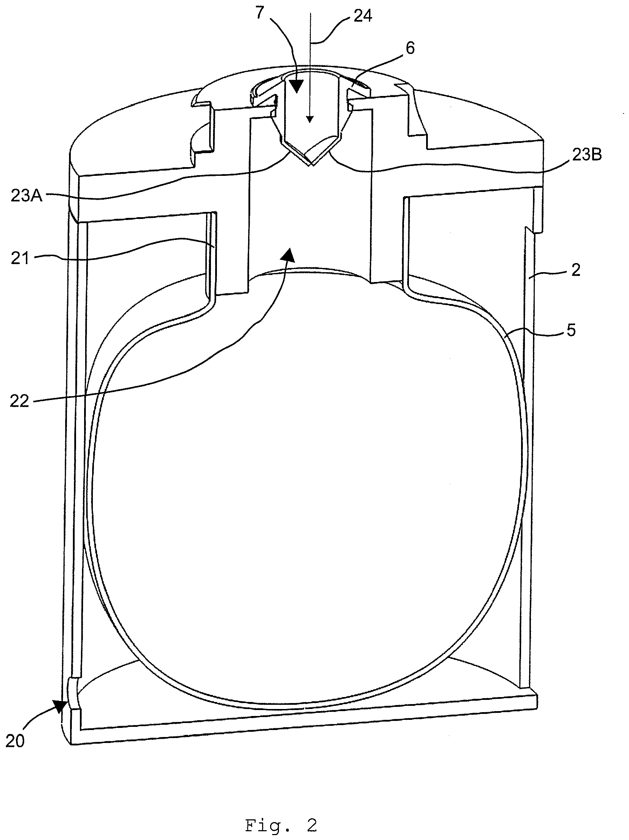

Figur 2 zeigt einen Schnitt durch einen Flüssigkeitstank 1 entsprechend der Figur 1. Das Gehäuse 3 weist dabei eine zusätzliche Öffnung 20 auf, welche zur Belüftung des Innenraums des Gehäuses 2 dient, damit das freigegebene Volumen beim kollabieren des Beutels 5 durch Entnahme der Flüssigkeit nicht zu einem Unterdruck im Inneren des Gehäuses führen kann.

Der Beutel 5 ist über einen Anschlussbereich 21 an einem Auslasskanal 22 des Gehäusedeckels 3 angeschlossen. Dieser Anschluss kann entweder durch elastische Deformation oder über Spannmittel, insbesondere Schellen oder Ringe abgedichtet werden.

Am oberen Ende des Auslasskanals 22 sitzt das Lippenventil 6 mit den angeordneten Ventillippen 23A, 23B. Wird nun durch die Öffnung 7 in Richtung 24 ein Öffnungsmittel eingeführt, durchstößt es die Ventillippen 23A, 23B und erlaubt eine Entnahme der Flüssigkeit aus dem Inneren des Beutels 5. Wird das Öffnungsmittel zurückgezogen, so führt ein Materialfluss aus dem Inneren des Beutels 5 zu einem Druck auf die Ventillippen 23A, 23B, wodurch diese verschlossen werden. Zur Bereitstellung des entsprechenden Materialdrucks kann der Beutel 5 über eine gewisse Elastizität bzw. Vorspannung, beispielsweise in Form eines Gummiballons verfügen.

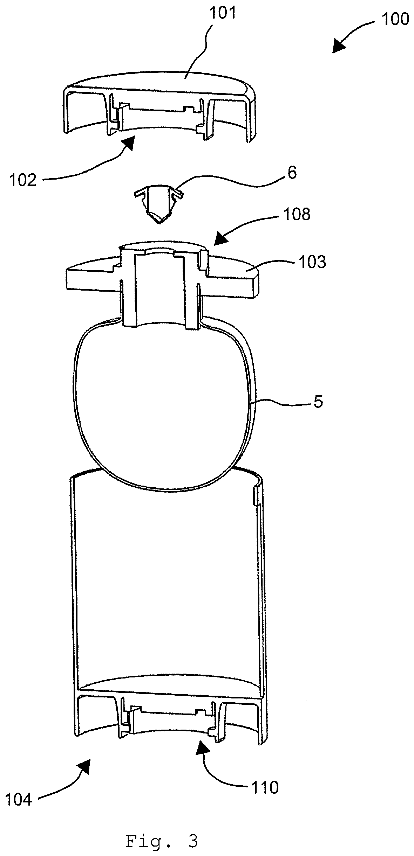

Figur 3 zeigt eine weitere Ausführungsform eines erfindungsgemäßen Flüssigkeitstanks 100 in Explosionsdarstellung. Gegenüber den oben beschriebenen Ausführungsformen verfügt die Ausführungsform des Flüssigkeitstanks 100 über einen zusätzlichen Verschlussdeckel 101, welcher über entsprechende Kupplungselemente 102 in die oben beschriebene mechanische Drehkupplung einbringbar ist.

Des Weiteren verfügt der Flüssigkeitstank 101 im Bereich seines Bodens 104 über ein entsprechendes korrespondierendes Kupplungselement 110 welches in seiner geometrischen Ausgestaltung derart gewählt ist, dass es in ein Kupplungselement 108 des Gehäusedeckels 103 in der oben beschriebenen Art eingreifen kann. Auf diese Weise können mehrere Flüssigkeitstanks 101 hintereinander angeordnet und aneinander befestigt werden. Die weiteren Bestandteile des Flüssigkeitstanks 101 entsprechend den oben beschriebenen Bestandteilen und sind mit gleichen Bezugszeichen beziffert.

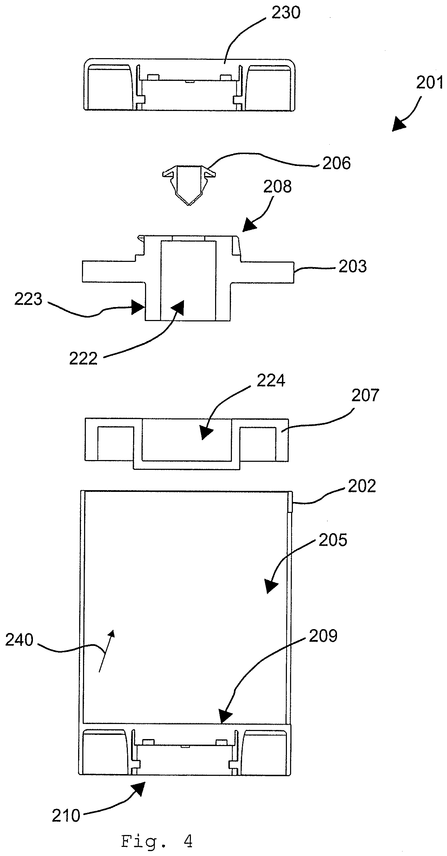

Figur 4 zeigt eine alternative Ausführungsform eines Flüssigkeitstanks 201 in explodierter Darstellung. Der Flüssigkeitstank 201 umfasst wiederum ein Gehäuse 202, einen Gehäusedeckel 203 sowie ein Lippenventil 206. Anstatt eines Beutels zur Bewahrung der Flüssigkeit ist nunmehr der Innenraum 205 des Gehäuses 202 zur Aufnahme einer Flüssigkeit ausgebildet. Bei der Entnahme der Flüssigkeit durch das Lippenventil 206, in dem die Flüssigkeit z.B. durch eine Saugpumpe abgesaugt wird, folgt ein passiv mitgeführter Folgekolben 207 dem verbleibenden Volumen der Flüssigkeit. Initial ist der Folgekolben 207 bei vollständig gefüllten Flüssigkeitstank im Bereich des unteren Gehäusebodens 209 angeordnet. Mit der Zeit bewegt sich der Kolben in Richtung 240 auf den Auslasskanal 222 des Gehäusedeckels 203 zu. Der untere Vorsprung 223 des Gehäusedeckels 203 kann dabei in eine Ausnehmung 224 des Folgekolbens 207 eintauchen, um eine vollständige Entleerung des Flüssigkeitstanks 201 so gut wie möglich zu ermöglichen.

Der Flüssigkeitstank 201 umfasst im Bereich des Gehäusedeckels 203 ebenfalls Kupplungsmittel 208, welche in korrespondierende Kupplungsmittel 210 eines anderen Flüssigkeitstanks entsprechend 201 eingreifen können, sodass die Flüssigkeitstanks 201 ebenfalls aneinander angeordnet werden können. Darüber hinaus kann auch ein Verschlussdeckel 230 entsprechend der oben beschriebenen Ausführungsform vorgesehen sein.

### Bezugszeichenliste:

- 1: Flüssigkeitstank
- 2: Gehäuse
- 3: Gehäusedeckel
- 4: Öffnung
- 5: Beutel
- 6: Ventil
- 8: Kupplungsmittel
- 9A,9B: Nuten
- 10: Drehrichtung
- 11: Kanal
- 12: Rastöffnung
- 13A, 13B: Kodierungselement
- 20: Öffnung
- 21: Anschlussbereich
- 22: Auslasskanal
- 23A, 23B: Ventillippen
- 100: Flüssigkeitstank
- 101: Verschlussdeckel
- 102: Kupplungselement
- 103: Gehäusedeckel
- 104: Boden
- 110: Kupplungselement
- 108: Kupplungselement
- 201: Flüssigkeitstank
- 202: Gehäuse
- 203: Gehäusedeckel
- 205: Innenraum
- 206: Lippenventil
- 207: Folgekolben
- 208: Kupplungsmittel
- 209: Gehäuseboden
- 210: Kupplungsmittel
- 222: Auslasskanal
- 223: Vorsprung
- 224: Ausnehmung
- 240: Bewegungsrichtung
- 230: Verschlussdeckel

## Patentansprüche

1. Flüssigkeitstank (1) für einen Zerstäuber für Flüssigkeiten, insbesondere einen elektrostatischen Zerstäuber, wobei der Flüssigkeitstank (1) einen Aufnahmeraum für eine Flüssigkeit und eine Begrenzungswand zur Begrenzung des Aufnahmeraums gegenüber der Umgebung umfasst,
wobei der Flüssigkeitstank (1) ein selbst schließendes Ventil (6) zum Anschluss am Zerstäuber umfasst, wobei mindestens ein Sensorelement zur Erfassung der Umgebungsparameter des Flüssigkeitstanks (1) und/oder der Umgebungsparameter der Flüssigkeit umfasst ist,
**dadurch gekennzeichnet, dass** das Sensorelement über ein Speicherelement zur Speicherung der Umgebungsparameter verfügt.

2. Flüssigkeitstank nach Anspruch 1, **dadurch gekennzeichnet, dass** die Begrenzungswand und/oder ein die Begrenzungswand umgebendes Gehäuse (2) aus isolierendem Material besteht, insbesondere eine elektrostatische Abschirmung ausbildet wobei das Material bevorzug eine Durchschlagfestigkeit von mindestens 30kV besitzt und/oder insbesondere eine Wandstärke von mindestens 1 mm aufweist.

3. Flüssigkeitstank nach einem der vorangegangenen Ansprüche, **dadurch gekennzeichnet, dass** die Begrenzungswand und/oder ein die Begrenzungswand umgebendes Gehäuse (2) eine Kontaktierung auf ein Bezugspotential gegenüber einer Hochspannung, z.B. gemeinsamer Minuspol bzw. ein gemeinsames Massepotential einer Hochspannungsquelle und eines Akkus, umfasst, wobei die Kontaktierung zumindest über einen Kontakt zur Verbindung mit dem elektrostatischen Zerstäuber verfügt.

4. Flüssigkeitstank nach einem der vorangegangenen Ansprüche, **dadurch gekennzeichnet, dass** der Flüssigkeitstank (1) Mittel zur Identifizierung (Kodierung) des Flüssigkeitstanks (1) umfasst, wobei diese Mittel insbesondere als mechanische Kodierung, bevorzugt zur Ausbildung eines Formschlusses, und/oder elektronische Kodierung, z.B. in Form einer RFID Markierung, insbesondere zur Übermittlung von Informationen über den Flüssigkeitstank (1) (lesen) und zur Aufnahme von Informationen über den Flüssigkeitstank (1) (schreiben), und/oder elektrische Kodierung, insbesondere zur Ausbildung von elektrischen Leitungswegen über Kontakte, ausgebildet sind.

5. Flüssigkeitstank nach einem der vorangegangenen Ansprüche, **dadurch gekennzeichnet, dass** die Begrenzungswand flexibel, insbesondere als kollabierbarer Beutel (5) ausgebildet ist.

6. Flüssigkeitstank nach einem der vorangegangenen Ansprüche, **dadurch gekennzeichnet, dass** die Begrenzungswand als Zylinder, vorzugsweise mit einem passiv geführten Kolbenboden ausgebildet ist.

7. Flüssigkeitstank nach einem der vorangegangenen Ansprüche, **dadurch gekennzeichnet, dass** mindestens ein weiterer Aufnahmeraum für eine weitere Flüssigkeit und eine Begrenzungswand zur Begrenzung des Aufnahmeraums gegenüber der Umgebung und dem ersten Aufnahmeraum umfasst ist.

8. Flüssigkeitstank nach einem der vorangegangenen Ansprüche, **dadurch gekennzeichnet, dass** mindestens eine Füllstandanzeige zur Anzeige des Füllstand der Flüssigkeit in dem Aufnahmeraum umfasst ist.

9. Flüssigkeitstank nach einem der vorangegangenen Ansprüche, **dadurch gekennzeichnet, dass** das selbst schließende Ventil (6) als Membran, Lippenventil (206), Kugelventil oder Federventil ausgebildet ist.

10. Flüssigkeitstank nach einem der vorangegangenen Ansprüche, **dadurch gekennzeichnet, dass** der Flüssigkeitstank (1) im Umgebungsbereich des Ventils (6) an einem Gehäuse (2) ein erstes mechanisches Kupplungselement (108), insbesondere ein Bajonettelement oder einen Drehriegelmechanismus umfasst und bevorzugt an einem dem Ventil (6) abgewandten Abschnitt des Gehäuses ein zweites mechanisches Kupplungselement (110) umfasst, dessen Geometrie korrespondierend verbindbar zum ersten mechanischen Kupplungselement (108) ausgebildet ist.

## Claims

1. Liquid tank (1) for an atomizer for liquids, in particular an electrostatic atomizer, the liquid tank (1) comprising a receiving space for a liquid and a delimiting wall for delimiting the receiving space with respect to the surrounding area, the liquid tank (1) comprising a self-closing valve (6) for connection to the atomizer, at least one sensor element for detecting the ambient parameters of the liquid tank (1) and/or the ambient parameters of the liquid being comprised, **characterized in that** the sensor element has a memory element for storing the ambient parameters.

2. Liquid tank according to Claim 1, **characterized in that** the delimiting wall and/or a housing (2) surrounding the delimiting wall consists of insulating material, in particular forms an electrostatic shielding, the material preferably having a dielectric strength of at least 30 kV and/or having in particular a wall thickness of at least 1 mm.

3. Liquid tank according to one of the preceding claims, **characterized in that** the delimiting wall and/or a housing (2) surrounding the delimiting wall comprises a contacting to a reference potential with respect to a high voltage, for example a common negative pole or a common ground potential of a high-voltage source and a storage battery, the contacting having at least one contact for connecting to the electrostatic atomizer.

4. Liquid tank according to one of the preceding claims, **characterized in that** the liquid tank (1) comprises means for identifying (coding) the liquid tank (1), this means being formed in particular as mechanical coding, preferably for forming an interlocking engagement, and/or electronic coding, for example in the form of an RFID marking, in particular for transmitting information about the liquid tank (1) (reading) and for receiving information about the liquid tank (1) (writing), and/or electrical coding, in particular for forming electrical line routes via contacts.

5. Liquid tank according to one of the preceding claims, **characterized in that** the delimiting wall is formed as flexible, in particular as a collapsible bag (5).

6. Liquid tank according to one of the preceding claims, **characterized in that** the delimiting wall is formed as a cylinder, preferably with a passively guided piston head.

7. Liquid tank according to one of the preceding claims, **characterized in that** at least one further receiving space for a further liquid and a delimiting wall for delimiting the receiving space with respect to the surrounding area and the first receiving space are comprised.

8. Liquid tank according to one of the preceding claims, **characterized in that** at least one filling level indicator for indicating the filling level of the liquid in the receiving space is comprised.

9. Liquid tank according to one of the preceding claims, **characterized in that** the self-closing valve (6) is formed as a diaphragm, a lip valve (206), a ball valve or a spring valve.

10. Liquid tank according to one of the preceding claims, **characterized in that** the liquid tank (1) comprises a first mechanical coupling element (108), in particular a bayonet element or a rotary bolt mechanism, in the surrounding region of the valve (6) on a housing (2) and preferably comprises a second mechanical coupling element (110), the geometry of which is formed correspondingly such that it can be connected to the first mechanical coupling element (108), on a portion of the housing that is facing away from the valve (6).

## Revendications

1. Réservoir de liquide (1) destiné à un atomiseur de liquides, notamment un atomiseur électrostatique, le réservoir de liquide (1) comprenant un espace de réception d'un liquide et une paroi de délimitation destinée à délimiter l'espace de réception par rapport à l'environnement, le réservoir de liquide (1) comprenant une soupape à fermeture automatique (6) destinée à être raccordée à l'atomiseur, au moins un élément de détection étant inclus qui est destiné à détecter les paramètres d'environnement du réservoir de liquide (1) et/ou les paramètres d'environnement du liquide,
**caractérisé en ce que**
l'élément de détection comporte un élément de mémorisation destiné à mémoriser les paramètres d'environnement.

2. Réservoir de liquide selon la revendication 1, **caractérisé en ce que** la paroi de délimitation et/ou un boîtier (2) entourant la paroi de délimitation est en un matériau isolant, formant en particulier un écran électrostatique, le matériau ayant de préférence une rigidité diélectrique d'au moins 30 kV et/ou ayant en particulier une épaisseur de paroi d'au moins 1 mm.

3. Réservoir de liquide selon l'une des revendications précédentes, **caractérisé en ce que** la paroi de délimitation et/ou un boîtier (2) entourant la paroi de limitation comprend une mise en contact à un potentiel de référence par rapport à une haute tension, par exemple un pôle moins commun ou un potentiel de masse commun d'une source de haute tension et d'un accumulateur, la mise en contact se faisant par le biais d'au moins un contact de liaison à l'atomiseur électrostatique.

4. Réservoir de liquide selon l'une des revendications précédentes, **caractérisé en ce que** le réservoir de liquide (1) comprend des moyens d'identification (codage) du réservoir de liquide (1), ces moyens étant conçus notamment sous la forme de codage mécanique, de préférence pour former une liaison par complémentarité de formes, et/ou de codage électronique, par exemple sous la forme d'une étiquette RFID, notamment pour transmettre des informations sur le réservoir de liquide (1) (lecture) et enregistrer des informations sur le réservoir de liquide (1) (écriture), et/ou d'un codage électrique, notamment pour former des chemins de conduction électrique par le biais de contacts.

5. Réservoir de liquide selon l'une des revendications précédentes, **caractérisé en ce que** la paroi de délimitation est souple, notamment est conçue sous la forme d'une poche repliable (5).

6. Réservoir de liquide selon l'une des revendications précédentes, **caractérisé en ce que** la paroi de délimitation est conformée en cylindre, pourvu de préférence d'une tête de piston à guidage passif.

7. Réservoir de liquide selon l'une des revendications précédentes, **caractérisé en ce qu'**au moins un autre espace de réception destiné à un autre liquide et une paroi de délimitation destinée à délimiter l'espace de réception par rapport à l'environnement et au premier espace de réception sont inclus.

8. Réservoir de liquide selon l'une des revendications précédentes, **caractérisé en ce qu'**au moins un indicateur de niveau de remplissage est inclus pour indiquer le niveau de remplissage du liquide dans l'espace de réception.

9. Réservoir de liquide selon l'une des revendications précédentes, **caractérisé en ce que** la soupape à fermeture automatique (6) est conçue sous la forme d'une membrane, d'une soupape à lèvre (206), d'une soupape à bille ou d'une soupape à ressort.

10. Réservoir de liquide selon l'une des revendications précédentes, **caractérisé en ce que** le réservoir de liquide (1) comprend, au niveau d'un boîtier (2) dans la zone entourant la soupape (6), un premier élément d'accouplement mécanique (108), notamment un élément à baïonnette ou un mécanisme de verrouillage rotatif, et comprend, de préférence au niveau de la portion du boîtier qui est à l'opposé de la soupape (6), un deuxième élément d'accouplement mécanique (110) dont la géométrie est conçue de manière correspondante pour permettre une liaison au premier élément d'accouplement mécanique (108).
